# FASCICULE DE BREVET EUROPEEN

(11) **EP 3 128 982 B1**
(45) Date de publication et mention de la délivrance du brevet: **14.06.2023**
(21) Numéro de dépôt: 15713773.8
(22) Date de dépôt: 07.04.2015
(51) Int. Cl.: A61F 13/514, A61F 13/62, A61F 13/84, B32B 5/26, B32B 7/00, B32B 7/12, B32B 5/02, B32B 5/14, B32B 7/08

(54) **NAPPE OU RUBAN À BOUCLES EN FORME DE STRATIFIÉ COMPORTANT DES MOTIFS IMPRIMÉS**
STREIFEN ODER ANSATZ MIT SCHLEIFEN IN FORM EINES LAMINATS MIT GEDRUCKTEN MUSTERN
STRIP OR LAP COMPRISING LOOPS, IN THE FORM OF A LAMINATE WITH PRINTED PATTERNS

(30) Priorité: 08.04.2014 FR 1400849
(43) Date de publication de la demande: 15.02.2017
(73) Titulaire: Aplix, 44850 Le Cellier (FR)
(72) Inventeur: MOINARD, Nathalie, 44980 Sainte-Luce-sur-Loire (FR); MARCHE, Thierry, 44450 La Chapelle- Basse-Mer (FR)
(74) Mandataire: Eidelsberg, Olivier Nathan
(86) Numéro de dépôt international: PCT/EP2015/057443
(87) Numéro de publication internationale: WO 2015/155152

(56) Documents cités:
- JP-A- 2013 121 428
- US-A1- 2008 102 725
- US-B2- 8 092 895

## Description

### DOMAINE TECHNIQUE

La présente invention se rapporte à une nappe ou un ruban à boucles, en forme de stratifié, pour former la partie boucles d'un auto agrippant à crochets et boucles, notamment pour des couches culottes et notamment pour former une bande disposée dans la partie avant de la couche culotte, au niveau de la ceinture en position centrale, bande classiquement appelée « landing zone », pour permettre la fermeture de la ceinture de la couche culotte par l'accroche de crochets issus de pattes disposées dans des parties d'extrémités latérales de la partie arrière de la couche culotte.

### ARRIERE PLAN DE L'INVENTION

On connaît déjà dans l'art antérieur des nappes ou des rubans en forme de stratifié de ce genre, comportant une couche textile en non-tissé formant support sur une face extérieure de laquelle est déposée en succession une couche d'encre pour former des motifs, par exemple décoratifs, puis une couche textile extérieure sous la forme d'un tricot comportant des boucles destinées à coopérer avec des crochets, la couche extérieure en forme de tricot étant fixée à la couche support et/ou à la couche d'encre par l'intermédiaire d'une couche de colle. Le tricot étant un tissu peu dense, il laisse voir de l'extérieur les motifs formés par la couche d'encre entre le tricot et la couche support de non tissé.

Ces stratifiés de l'art antérieur présentent plusieurs inconvénients.

D'une part, ils sont compliqués à fabriquer, ce qui limite la possibilité de les fabriquer à grande échelle et à bas coût, caractéristiques particulièrement recherchées dans le domaine des couches culottes jetables où les cadences de fabrication peuvent dépasser les 1000 couches/min. En particulier, l'encre a tendance à souiller les rouleaux de convoyage des rubans, entraînant leur encrassement rapide et la nécessité d'avoir à les nettoyer avec une grande fréquence, ce qui limite nettement la vitesse de production du produit. En outre, la fixation par collage du tricot est rendue difficile par la présence de la couche d'encre. D'autre part, ces stratifiés de l'art antérieur ont une fâcheuse tendance au délaminage lors de l'utilisation, notamment lors d'ouvertures et fermetures répétées d'une couche culotte.

Dans US 8092895, il est décrit un stratifié comportant une couche de non-tissé, sur laquelle est disposée une couche d'encre et un tricot extérieur formant des boucles collé au non-tissé.

En outre, une solution y est apportée au problème du souillage des rouleaux de convoyage par l'usure en prévoyant un prétraitement de la surface supérieure du non-tissé. Ce prétraitement a pour effet de maintenir l'encre au dessus du non-tissé en en diminuant sa capacité à se déposer sur les rouleaux.

Cette étape de prétraitement implique soit l'utilisation de techniques compliquées soit l'adjonction de couches supplémentaires destinées à recevoir l'encre.

On connaît également de US-A-2008/0102725 un stratifié comportant une couche intérieure de papier sur laquelle sont disposés des motifs, et une couche extérieure de non-tissé liée par hydroenchevêtrement à la couche intérieure de papier. La couche extérieure de non tissé a des propriétés d'absorption pour une utilisation dans des chiffons d'essuyage. Le procédé de fabrication de ce stratifié est compliqué et l'hydroenchevêtrement pour la liaison non tissé-papier nuit en outre à la qualité, notamment la netteté, des motifs.

On connaît également de JP-A-2013-121428 un stratifié comportant une couche de non-tissé à boucle et une couche de non-tissé de support. De l'encre est appliquée sur le non-tissé à boucle pour former des motifs. Outre que la technique de fabrication est compliquée, les motifs se voient mal de l'extérieur, l'encre s'éparpillant dans le non-tissé bouclé de manière diffuse.

### OBJET ET RESUME DE L'INVENTION

La présente invention vise à surmonter les inconvénients de l'art antérieur, en proposant un stratifié formant nappe ou ruban à boucles d'un système auto agrippant à crochets et boucles, notamment pour une couche culotte, qui, tout en assurant une bonne visibilité des dessins ou motifs imprimés du stratifié, peut être fabriqué à grande vitesse et résiste bien au délaminage en utilisation, notamment lors de cycles répétés d'ouvertures et de fermetures d'une couche culotte.

Le stratifié selon l'invention est défini à la revendication 1.

En prévoyant ainsi de sélectionner le non-tissé et/ou l'encre de manière à ce que l'encre formant la couche d'impression des motifs décoratifs soit totalement reçue à l'intérieur de la couche de non-tissé, on améliore nettement la vitesse de fabrication du stratifié et sa résistance au délaminage. En effet, l'encre étant maintenant, pour sa plus grande partie, de préférence sa totalité, reçue à l'intérieur du non-tissé support, elle n'interfère plus avec le processus de fixation de l'élément textile supérieur, par exemple avec l'encollage. Il en résulte d'ailleurs la possibilité nouvelle d'employer lors de ce processus de fixation une étape de soudure, alors même que dans l'art antérieur, ce type de processus était impossible en raison de la présence de la couche d'encre. La fixation est ainsi simplifiée car il n'est plus nécessaire de prévoir des colles complexes à adapter pour une fixation sur une couche d'encre. En outre, l'encre n'est plus susceptible de souiller les rouleaux de convoyage, épargnant ainsi des temps d'arrêt nécessaires à leur nettoyage.

En outre, la fixation de l'élément textile supérieur est améliorée, notamment par la possibilité maintenant existante d'utiliser la soudure, notamment le calandrage, de sorte que le stratifié est moins susceptible de se délaminer, notamment en tant que « landing zone » (ou « bande de confort ») pour une couche culotte.

La totalité de la couche d'encre est reçue dans la au moins une couche de non tissé et affleure à la surface supérieure de la au moins une couche de non tissé.

Suivant un mode de réalisation préféré de l'invention, la fixation de la au moins une couche extérieure textile à boucles à la au moins une couche de non-tissé formant support est réalisée par soudure.

De préférence, cette soudure est réalisée sans apport de matière, notamment par un calandrage thermique et/ou mécanique ou par une soudure à ultrason.

Suivant un mode de réalisation de l'invention, la au moins une couche textile extérieure à boucles est un tricot.

Suivant un autre mode de réalisation particulièrement favorable, la au moins une couche textile extérieure à boucles est un non-tissé, notamment un non-tissé cardé consolidé, plus particulièrement un non tissé cardé calandré et/ou hydrolié ou analogue.

Suivant encore un autre mode de réalisation favorable, la couche textile à boucle est constituée d'un amas de fibres ou filaments non consolidés en une couche de non tissé.

Suivant un perfectionnement avantageux, il est prévu des moyens pour bloquer la migration de l'encre dans la au moins une couche de non tissé pour la maintenir dans la partie supérieure du non tissé, notamment en affleurement à la face extérieure du non tissé, et ainsi assurer une bonne visibilité de l'encre à partir du côté extérieur, ou du dessus.

En particulier, suivant un mode de réalisation avantageux, la au moins une couche de non-tissé comporte au moins deux couches empilées l'une sur l'autre, une couche supérieure, du côté extérieur, favorisant la migration en son sein de l'encre des motifs décoratifs de la couche d'impression pour ainsi recevoir l'encre, et une couche inférieure, du côté intérieur, moins favorable à la migration de cette encre et notamment une couche bloquant la migration de l'encre pour former les moyens de blocage de la migration de l'encre.

Suivant un mode de réalisation particulièrement favorable, la au moins une couche de non-tissé est constituée d'une couche supérieure en Spunbond et d'une couche inférieure de blocage de type Meltblown, pour ainsi former un non tissé SM (Spunbond-Meltblown) l'épaisseur de la couche Spunbond étant suffisante pour permettre la migration de sensiblement la totalité de la couche d'encre en son sein avant que cette dernière ne soit arrêtée par la couche de type Meltblown, le non tissé étant notamment un SMS, un SMMS, SSMMS et analogue.

Suivant un mode de réalisation préféré, le stratifié est dépourvu ou sensiblement dépourvu d'encre sur ses faces extérieure et intérieure lorsque l'on réalise sur ces deux faces le test de la bande adhésive (défini ci après).

De préférence, la couche de non tissé support (SMS par exemple) présente, notamment avant calandrage avec la couche textile extérieure à boucles (cardé calandré par exemple), une perméabilité à l'air supérieure à 1000 l/m²/s, préférentiellement supérieure à 1700 l/m²/s.

La présente invention se rapporte également à une couche culotte incorporant un stratifié suivant l'invention, notamment dans la partie centrale de la partie avant, au niveau de la ceinture pour y former la « landing zone » ou « bande confort ».

### DESCRIPTION SUCCINTE DES DESSINS

A titre d'exemple, on décrit maintenant des modes de réalisation préférés de l'invention en se reportant aux dessins, dans lesquels :
- la figure 1: est une vue en coupe schématique d'un premier mode de réalisation d'un stratifié suivant l'invention comportant, comme élément textile à boucles, un tricot, la liaison étant réalisée par calandrage ;
- la figure 2: est une vue en coupe schématique d'un deuxième mode de réalisation de l'invention dans lequel l'élément textile à boucles est un non-tissé, notamment cardé consolidé, la liaison étant réalisée par calandrage ;
- la figure 3: est une vue en coupe d'encore un autre mode de réalisation de l'invention réalisé avec un élément textile à boucles en forme de tricot, la liaison étant réalisée par une couche de colle ;
- la figure 4: est une vue en coupe schématique d'encore un autre mode de réalisation comportant, comme élément textile un non-tissé, notamment cardé calandré, la liaison étant réalisée par une couche de colle ;
- la figure 5: représente schématiquement en perspective une couche culotte incorporant dans sa partie frontale, au niveau central de la ceinture, un stratifié pour former la landing zone, ce stratifié pouvant être n'importe lequel de ceux décrits dans la présente demande comme étant suivant l'invention ;
- la figure 6: est une vue schématique de côté d'une installation pour la fabrication d'un stratifié suivant l'invention, notamment pour la fabrication d'un stratifié suivant la figure 2 ;
- la figure 7A: est la vue de dessus d'un échantillon d'un non tissé SMS imprimé utilisé, suivant un mode de réalisation de l'invention, en tant que la couche de non tissé formant support, avant l'application de la bande d'adhésif pour le test dit de la bande adhésive ;
- la figure 7B: est la vue de dessus de l'échantillon de la figure 7A, après application et enlèvement de la bande adhésive ;
- la figure 7C: est la vue de dessus de la bande adhésive après qu'elle ait été appliquée sur l'échantillon et enlevée de ce dernier ;
- la figure 8A: est la vue de dessus (c'est à dire du côté des boucles, ou du cardé consolidé) d'un échantillon d'un stratifié comportant le non tissé SMS imprimé de la figure 7A et un non tissé cardé calandré sur le SMS,
- La figure 8B: est la vue de dessus du SMS seul de l'échantillon de la figure 8A, après délaminage du cardé calandré ; et
- la figure 8C: est la vue de dessus du cardé seul après qu'il ait été enlevé par délaminage du SMS de l'échantillon.

### DESCRIPTION DETAILLEE DE MODES DE REALISATION

Pour fermer une couche culotte C de manière temporaire, on utilise un dispositif à crochets et boucles. En général, les boucles sont disposées sur la partie frontale de la ceinture de la couche culotte, tandis que des pattes latérales comportant des crochets font saillie de la partie de dos de la ceinture pour venir s'accrocher dans les boucles de la partie frontale. Cependant, on pourrait prévoir l'inverse, en disposant des boucles dans les parties latérales et des crochets dans la partie frontale.

Pour réaliser la partie à boucles, et notamment dans la partie frontale appelée bande confort, on utilise un stratifié en forme de ruban comportant au moins une couche de support en un non-tissé et un élément à boucles. La couche de non-tissé comporte une face intérieure destinée à être du côté intérieur, c'est à dire du côté du porteur de la couche culotte, notamment en y étant fixée, tandis que la couche extérieure se trouve à l'opposé, c'est à dire du côté extérieur par rapport au porteur de la couche culotte. Cette face extérieure est destinée à être recouverte par un élément textile comportant des boucles.

De manière générale, dans la présente demande, on entend par supérieure le côté extérieur d'un élément et par inférieur le côté intérieur d'un élément. Le côté extérieur est le côté où se trouvent les boucles et par lequel les crochets viennent s'accrocher aux boucles.

La fixation des deux strates (à savoir le non tissé support et l'élément textile à boucles) du stratifié est réalisée par exemple par encollage. Cependant, on peut à la place prévoir une soudure, notamment par calandrage mécanique et/ou thermique ou par ultrasons.

Cependant, avant l'encollage ou la soudure, il est appliqué sur la face extérieure du non-tissé, une couche d'encre d'impression pour la formation de motifs ou dessins.

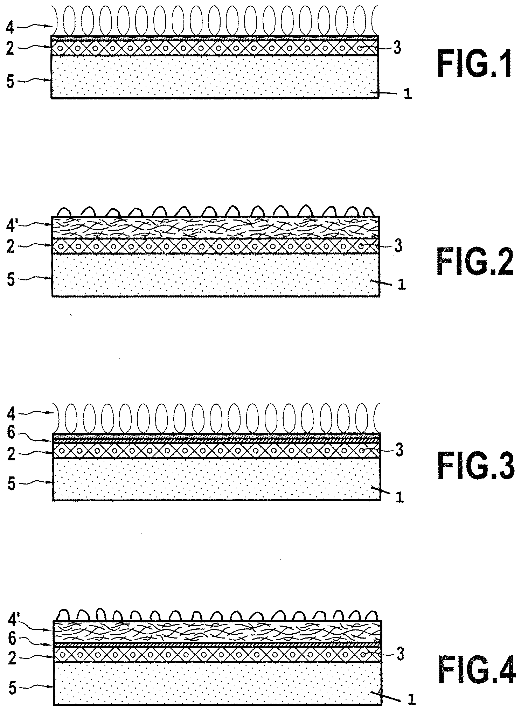

A la figure 1, il est représenté un premier mode de réalisation de l'invention. La couche 1 support de non-tissé (qui peut d'ailleurs en soi former une partie de la couche culotte) comporte une région 2 supérieure, qui se trouve du côté extérieur de la couche 1 support. Cette couche 2 est en un matériau dans lequel l'encre d'une couche 3 d'impression appliquée sur la couche 1 support a migré pour y être incorporée sensiblement en totalité, en affleurant à la surface supérieure ou extérieure du non tissé 1. Ainsi, la couche 3 d'encre n'interfère plus avec le processus de fixation d'un élément textile portant les boucles.

A la figure 1, cet élément textile à boucles est constitué d'un tricot 4 à boucles. Le tricot 4 à boucles est fixé par calandrage à la face extérieure du non tissé 1. Comme l'encre a totalement migré dans le non tissé 1, elle ne gêne pas le processus de calandrage, qui peut ainsi être facilement mis en œuvre. En outre, il n'est plus nécessaire de prévoir soit des encres soit des colles complexes et coûteuses, soit les deux, permettant l'encollage malgré la présence d'encre.

Pour éviter que la couche 3 d'encre ne pénètre jusqu'au fond du non-tissé 1 support, on prévoit de réaliser le non tissé sous la forme de plusieurs couches superposées, avec notamment une couche 5 intermédiaire anti-migration dans laquelle l'encre n'a pas de faculté ou n'a que des facultés de migration faibles par rapport à la couche supérieure ou extérieure dans laquelle elle a des facultés de migration importantes. Ainsi, comme représenté à la figure 1, la couche d'encre est arrêtée par la couche 5 intermédiaire qui est de structure et/ou de matière différente de la couche 2 supérieure.

En particulier, on peut définir la capacité d'un couple encre-couche de non tissé à intégrer l'encre dans la couche de non tissé par le test suivant dit de la bande adhésive :
1. On sélectionne un échantillon constitué d'au moins une couche de non tissé sur laquelle a été appliquée de l'encre. En particulier, l'échantillon peut être sous la forme d'une bande de 10 cm de long sur 3 cm de large ;
2. On le place sur un support plan, dans les conditions normales de température et pression (25°C et 1 atm) ;
3. On dépose sans pression sur une face de l'échantillon une bande adhésive de type « Scotch » (marque déposée), par exemple un Scotch (marque déposée) de la société 3M référencé 313, de préférence fabriqué depuis moins de trois mois, en laissant libre une zone de préhension ;
4. On applique ensuite, à vitesse constante (environ 20cm/s), sur toute la largeur de la bande adhésive de l'échantillon et en un aller une pression avec un rouleau applicateur de 2kg et d'une largeur de 49mm de manière à presser la bande adhésive sur l'échantillon 5. On tire ensuite la bande adhésive pour la séparer de l'échantillon (environ 40cm/s, avec un angle de tirage de 50° environ). On valide cette étape si les boucles ou de manière générale l'échantillon ne présentent pas de déchirure ou de rupture dans l'épaisseur ou encore de défibrillation.
6. On contrôle alors visuellement la zone de l'adhésif ayant été en contact avec les zones imprimées de l'échantillon pour évaluer la quantité d'encre ayant été transférée de l'échantillon à la bande adhésive pour en particulier constater l'absence ou la présence d'encre sur la bande adhésive.
7. Ensuite, on détermine la surface apparente du ou des motifs d'encre S apparaissant sur la surface de l'échantillon sur laquelle a été appliquée la bande adhésive et la surface apparente S' du ou des motifs formés par l'encre sur la bande adhésive et on calcule le rapport S'/S sous la forme d'un pourcentage donnant une mesure de l'intégration de l'encre au non tissé, un grand pourcentage correspondant à une faible intégration et un petit pourcentage correspondant à une bonne intégration.

Ce test peut être réalisé aussi bien sur la face extérieure du non tissé ou du stratifié que sur sa face intérieure opposée du non tissé ou du stratifié.

Par exemple, suivant l'invention on entend par l'expression « le stratifié ou l'élément non tissé est dépourvu ou sensiblement dépourvu d'encre » un stratifié ou un élément non tissé pour lequel on obtient dans le présent test un rapport inférieur à 10%, de préférence inférieur à 5%, encore plus préférablement inférieur à 1%, par exemple inférieur à 0,5%.

Pour calculer les surfaces apparentes S et S', on peut notamment procéder de la manière suivante :
1. on positionne la bande adhésive comprenant des motifs d'encre sur une feuille de papier, par exemple une feuille de papier de couleur blanche, du fait de la couleur du non tissé support, de dimension 21x29,7mm et de 80g/m², de sorte que l'encre soit emprisonnée entre la feuille de papier et la bande adhésive.
2. on positionne sur la feuille de papier à une position voisine de la bande adhésive avec de l'encre, l'échantillon de sorte que les motifs soient orientés du côté opposé à la feuille de papier.
3. On utilise une nouvelle bande adhésive que l'on applique sur l'échantillon de sorte qu'il soit fixé à la feuille et que les motifs soient totalement recouverts de la nouvelle bande adhésive.
4. On trace sur la feuille deux zones (de formes et dimensions identiques) de sorte qu'elles soient centrées respectivement d'une part sur le ou les motifs de la bande adhésive comprenant de l'encre et d'autre part le ou les motifs de l'échantillon.
5. on numérise (en noir et blanc ou en couleur) à haute définition par exemple de 600x600PPP (nombre de points par pouce ou encore DPI pour dots per inch en anglais), la feuille de papier de sorte que l'échantillon et la bande adhésive puissent être traités numériquement.
6. on calcule les surfaces apparentes de la zone comprenant l'adhésif avec l'encre et la zone comprend l'échantillon recouvert de la deuxième bande d'adhésif par un traitement numérique en utilisant un logiciel spécialisé, par exemple « ImageJ ».
7. on transforme l'image numérisée (c'est à dire la zone comprenant la bande adhésive avec l'encre et la zone comprenant l'échantillon) de sorte qu'elle présente uniquement deux types de pixels, un pixel noir ou un pixel blanc.
8. On compte le nombre de pixels noirs pour déterminer les surfaces apparentes S et S'.

Dans le cas où l'utilisateur observe une absence d'encre à l'ceil nu sur la bande adhésive, on considère que la surface S' est nulle et que l'intégralité de l'encre a migré dans le non tissé.

Pour l'acquisition et le traitement de l'image, on pourrait également utiliser un système de vision numérique de marque Cognex, modèle « système de vision insight 7000 », comprenant, par exemple, un objectif dont la longueur focale de la lentille est de 12 mm, et le logiciel de traitement dédié « Easybuilder » (marque déposée). Avec ce système de vision numérique, on peut, par exemple, compter le nombre de pixels noirs pour déterminer les surfaces apparentes S et S' après avoir réalisé une transformation similaire à celle de l'étape 7 précédemment décrite, ou encore, par exemple, calculer la moyenne en niveau de gris des surfaces S et S', sans que celles-ci aient été transformées selon l'étape 7 précédemment décrite. Selon encore une autre méthode utilisant le système de vision numérique cité ci-dessus, on peut comparer le niveau de gris de la surface apparente du ou des motif(s) d'encre S apparaissant sur la surface de l'échantillon sur laquelle a été appliquée la bande adhésive avec le niveau de gris de la surface apparente du ou des motif(s) d'encre S" apparaissant sur la surface de l'échantillon sur laquelle, d'une part, a été appliquée une première bande adhésive qui a été ensuite retirée, d'autre part, sur laquelle on a ajouté une deuxième bande adhésive.

On choisit ainsi une couche 2 supérieure présentant une bonne intégration de l'encre (rapport ci dessus égal à un faible pourcentage), et une couche intermédiaire présentant une faible intégration (rapport ci dessus égal à un pourcentage élevé).

Comme on le voit à la figure 1, l'encre 3 ayant cessé de migrer vers le fond du non-tissé en raison de la couche 5 intermédiaire de blocage, on voit toujours bien de l'extérieur les motifs. Dans le même temps, la couche d'encre ne gêne pas à la fixation du tricot. En outre, le calandrage étant sans apport de matière, il ne participe pas à l'opacité du stratifié, et notamment ne participe pas à la diminution de la visibilité des motifs imprimés à travers le tricot 4.

On obtient ainsi un système stratifié particulièrement rapide à fabriquer, qui résiste bien au délaminage et qui présente une bonne visibilité des motifs imprimés.

A la figure 2, il est représenté un autre mode de réalisation, dans lequel l'élément textile à boucles est un non-tissé 4', de préférence cardé calandré. Le non tissé 1 support est identique à celui du mode de réalisation représenté à la figure 1. La couche 3 d'encre n'a pas totalement migré au sein du non tissé 1 support. Bien qu'une majeure partie, notamment supérieure à 70%, notamment supérieur à 90%, préférentiellement supérieur ou égal à 95% de la couche d'encre se trouve au sein du non-tissé 1 support, une partie de la couche d'encre n'a pas migré dans le non tissé support. En revanche cette partie restante de la couche d'encre a migré dans le non tissé 4', de sorte qu'entre le non tissé 4' à boucles et le non tissé 1 support, il n'y a pas d'encre ou sensiblement pas d'encre présente susceptible de gêner le processus de fixation mutuelle des deux non tissés. Ce processus peut ainsi être réalisé par calandrage, sans apport de matière, ce qui favorise une bonne visibilité des motifs définis par la couche d'encre à travers le non tissé 4' cardé calandré.

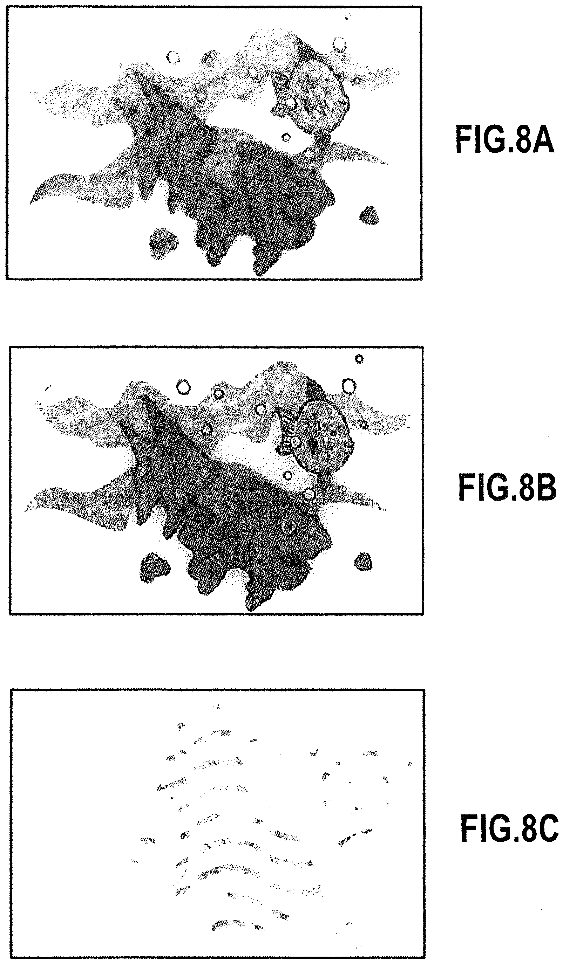

A la figure 8A, il est représenté un échantillon d'un stratifié correspondant au mode de réalisation de la figure 2, le non tissé support imprimé étant constitué d'un SMS, tandis que le non tissé à boucle est un non tissé cardé calandré.

Plus particulièrement, la couche de non tissé support (SMS par exemple) présente, avant calandrage avec la couche textile extérieure à boucles (cardé calandré) une perméabilité à l'air supérieure à 1000 l/m²/s, préférentiellement supérieure à 1700 l/m²/s mesurée selon la norme européenne et la norme française NF EN ISO 9237 Textiles de « détermination de la perméabilité de l'air des étoffes » avec un indicateur de pression ou manomètre de 200Pa.

Plus particulièrement, le stratifié présente une perméabilité à l'air supérieure à 500 l/m²/s et inférieure à 2400 l/m²/s mesurée selon la norme européenne et la norme française NF EN ISO 9237 Textiles de « détermination de la perméabilité de l'air des étoffes » avec un indicateur de pression ou manomètre de 200Pa.

Une séparation à la main (délaminage) a été effectuée et on obtient d'une part le non tissé SMS représenté à la figure 8B en vue de dessus et le non tissé cardé calandré représenté à la figure 8C. Comme on peut le voir à la figure 8C, de l'encre est présente sur le cardé calandré délaminé, principalement dans les anciennes zones de soudure du non tissé cardé au SMS.

Aux figures 3 et 4, il est représenté des modes de réalisation identiques respectivement à ceux des figures 1 et 2, dans lesquels la fixation des deux éléments respectivement support et à boucles est réalisée par une couche 6 de colle, à la place du calandrage.

Dans la présente demande, on entend par non tissé un produit obtenu à l'issue de la formation d'une nappe de fibres et/ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de ruban ou nappe de fibres et/ou filaments qui sont entrelacées d'une manière non uniforme, irrégulière ou au hasard. Un non-tissé peut avoir une structure de couche unique ou une structure à couches multiples. Un non-tissé peut également être réuni à un autre matériau pour former un stratifié. Un non-tissé peut être réalisé à partir de différents matériaux synthétiques et/ou naturels. Les matériaux naturels à titre d'exemple sont des fibres de cellulose, telles que coton, jute, lin et analogue et peuvent également inclure des fibres de cellulose retraitées, telles que la rayonne ou la viscose. Les fibres naturelles pour un matériau non-tissé peuvent être préparées en utilisant divers procédés tels que le cardage. Des matériaux synthétiques à titre d'exemple comportent, mais sans s'y limiter, des polymères thermoplastiques synthétiques, qui sont connus pour former des fibres qui incluent, sans s'y limiter, les polyoléfines, par exemple le polyéthylène, polypropylène, polybutylène et analogue ; le polyamide, par exemple le polyamide 6, polyamide 6.6, polyamide 10, polyamide 12 et analogue ; des polyesters, par exemple des polyéthylènes téraphthalates, des polybutylènes téréphtalates, des acides polylactiques et analogues, des polycarbonates, des polystyrènes, des élastomères thermoplastiques, des vinyles polymères, des polyuréthanes et des mélanges et des co-polymères de ces derniers.

On entend notamment par non tissé, comme suivant la définition donnée par l'INDA (Association of Non Woven Fabrics Industry) et l'EDANA (Association Européenne des Articles à Usage Unique et des Non tissés) à l'Organisation Internationale de Normalisation (ISO), une feuille de fibres, de filaments continus ou de fils coupés de toute nature et/ou origine qui ont été formés en une nappe par tout moyen et liés entre eux par tout moyen, à l'exception du tissage et du tricotage. Les feutres obtenus par un procédé de mouture ou broyage en voie humide, notamment les papiers, ne sont pas des non-tissés.

Les nappes obtenues par voie humide, dites « wetlaid » sont des non tissés à condition qu'elles contiennent un minimum de 50% de fibres d'origine synthétique ou d'origine autre que végétale ayant un rapport longueur sur diamètre égal ou supérieur à 300, ou un minimum de 30% de fibres synthétiques ayant un rapport longueur sur diamètre égal ou supérieur à 600, et une densité maximum apparente de 0,40 g/cm³.

Pour réaliser la soudure, entre l'élément textile et le support non-tissé, on peut réaliser la liaison suivant des motifs de toutes formes, par exemple continue, discontinue, en forme de vague, etc. Ce motif de liaison peut être réalisé en utilisant la chaleur, la pression, l'ultrason, ou des combinaisons de ces derniers. Par exemple, et de manière préférentielle, un non-tissé peut être lié en faisant passer le ruban non-tissé dans un interstice formé par un rouleau de calandrage chauffé comportant une gravure de liaison et un autre rouleau, de sorte que des îlots de la gravure forment des zones de liaison sur le ruban non-tissé. Cette description et définition du non-tissé s'appliquent aussi bien au non-tissé formant support, qu'au non-tissé pour le matériau à boucles.

Pour déposer la couche d'encre, on peut utiliser tout procédé classique, notamment la flexographie, l'impression par jet d'encre, la rotogravure, la sérigraphie, l'héliographie, et analogues. L'impression peut être mono couleur ou à couleurs multiples.

De préférence, l'encre est une encre non adhésive, c'est à dire une encre qui n'est pas capable de réaliser une liaison suffisante entre l'élément textile à boucles et le non tissé support.

Une encre est constituée d'un mélange de trois composants : une matière colorante, notamment un pigment ou un colorant ; un véhicule formant la phase fluide de l'encre, par exemple d'un mélange de polymères, de diluants et/ou de solvant ou encore d'eau ; et des additifs, tels que agents dispersants, anti mousse etc. permettant d'optimiser les caractéristiques de l'encre. Comme mélange de polymères, on peut utiliser des mélanges par exemple jusqu'à 50% de divers acétates, tels que acétate d'éthyle, acétate de N propyle, acétate d'iso propyle, acétate de N butyle, et leurs mélanges, jusqu'à 10% d'alcool.

On peut utiliser des pigments organiques ou minéraux, par exemple des colorants diazoïques, des colorants anthraquinone, xanthène, azine et analogues, dioxyde de titane, noir de carbone, oxydes de fer, oxyde de chrome et analogues.

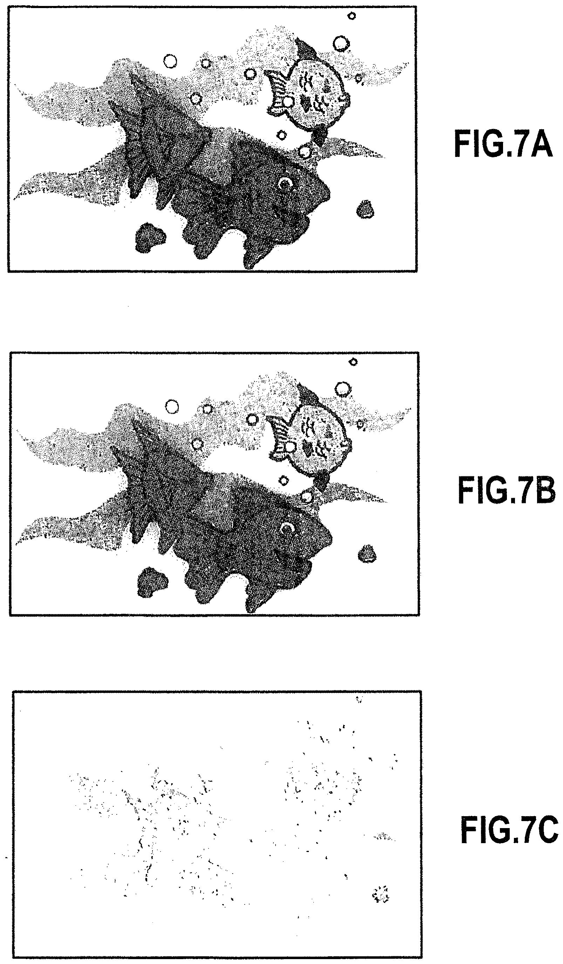

Aux figures 7A et 7B, on a représenté des vues de dessus respectivement d'un échantillon d'une couche de non tissé constituée d'un SMS imprimé, respectivement avant application de la bande d'adhésif pour réaliser le test de la bande adhésive et après application et enlèvement de la bande d'adhésif. A la figure 7C, on a représenté la vue de dessus de la bande d'adhésif appliquée et enlevée de l'échantillon de la figure 7A.

Comme on peut le voir notamment aux figures 7A à 7C, un petit pourcentage uniquement de l'encre n'a pas été intégré dans le non tissé SMS et apparaît sur la face intérieure de la bande adhésive. Le rapport de la surface d'impression sur la bande adhésive sur celle du non tissé est de l'ordre de 1,5% selon la méthode décrite précédemment.

Ci après, on donne, uniquement à titre d'illustration de l'invention et de manière non limitative, un exemple de couples de non tissé support-encre ou de trinômes non tissé support - encre - non tissé à boucles suivant l'invention.

### Exemple

Comme non tissé support, on prend un SMS de masse surfacique égale à 13g/m², dont les filaments des couches de Spunbond ont un diamètre moyen égal à 15 micromètres et les filaments de la couche de Meltblown ont un diamètre moyen de 6 micromètres, tandis que l'encre est constituée d'un mélange d'une résine nitrocellulose (1%), de Polyacetate (12%), d'Ethanol (47%) et de pigments (5%) .

On applique alors le test de la bande adhésive et trouve un rapport de surface imprimée de 1,54%.

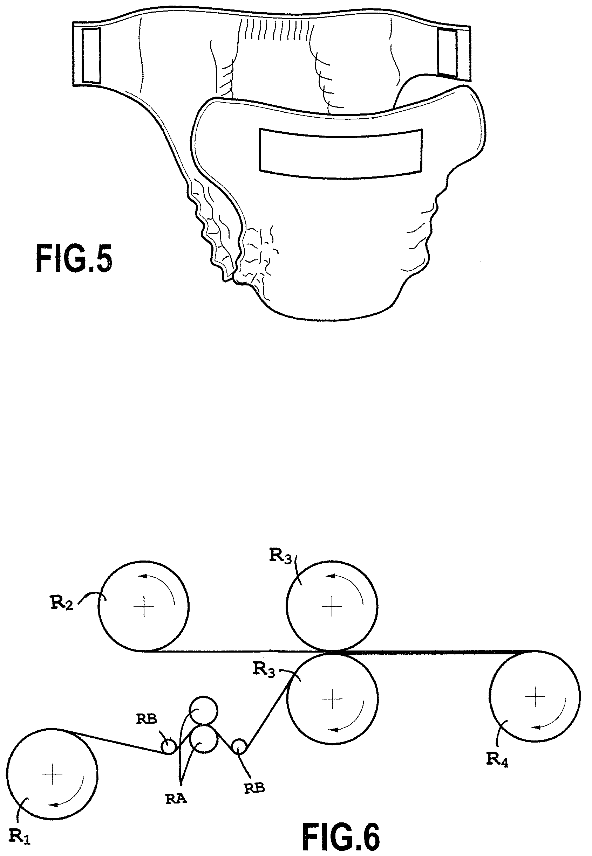

A la figure 6, il est représenté un schéma de principe d'une installation pour la fabrication d'un stratifié suivant la Figure 2.

Cette installation représente en ligne à la fois l'impression du SMS et le calandrage du cardé calandré sur le SMS imprimé. En variante de réalisation, on pourrait envisager de séparer les deux étapes de fabrication.

Selon l'exemple représenté à la figure 6, le rouleau R1 est agencé de sorte à dérouler le SMS non imprimé pour qu'il soit imprimé par les rouleaux RB, les rouleaux RA ayant pour fonction d'impartir de la tension dans le SMS. L'encre du SMS imprimé est ensuite séchée, notamment au moins une partie du véhicule formant la phase fluide de l'encre est évaporée par des moyens adaptés non représentés.

Le rouleau R2 est agencé de sorte à déroulé le cardé calandré de manière à ce que le cardé calandré et le SMS imprimé soient liés par calandrage thermique par les rouleaux R3 (dont l'un comprend une gravure) pour former le stratifié suivant l'invention. Le rouleau R4 est agencé pour enrouler le stratifié obtenu.

Suivant la présente invention, on entend par boucle un filament et/ou une fibre qui comprend deux extrémités chacune solidaire du support en un point respectif du support ou en un même point du support. Une boucle peut également être formée de plusieurs filaments ou fibres solidarisés entre eux et dont au moins deux d'entre eux sont solidarisés au support, en un point ou en deux points respectifs distincts.

On entend par non tissé à boucles un non tissé faisant boucles après liaison au support.

De manière générale, les fibres et les filaments diffèrent principalement par leur longueur et par leur procédé de fabrication.

On entend par filaments les éléments unitaires, de très grandes longueurs vis à vis du diamètre dans lequel s'inscrit leur section, extrudés de manière continue pour former directement une nappe de non tissé qui peut être ensuite consolidée par thermo-liage ou tout autre moyen pour permettre l'atteinte des performances souhaitées et/ou leur transport. De préférence, les filaments présentent une longueur supérieure à 120mm.

On entend par fibre le terme générique pour désigner une matière textile ou un élément de matière textile de longueur réduite, inférieure à la longueur des filaments, et susceptible d'être filée et/ou utilisée dans la réalisation de non tissés. On distingue deux types de fibres, les fibres courtes formées de matière discontinue de faible longueur inférieure à 70mm (préférentiellement de 25mm à 60 mm) et les fibres longues formées de manière discontinue de grande longueur supérieure à 70 mm (préférentiellement de 80 mm à 120 mm).

A la différence des filaments qui sont consolidés directement après avoir été extrudés, les fibres sont orientées et organisées en nappe lors d'une étape de cardage bien connue de l'homme du métier. Cette nappe peut être ensuite consolidée par thermo-liage ou tout autre moyen pour permettre l'atteinte des performances souhaitées et/ou leur transport.

## Revendications

1. Stratifié pour former un ruban ou une nappe à boucles, notamment la bande confort d'une couche culotte, comportant :
un élément non-tissé comportant au moins une couche (1) de non-tissé sur une face supérieure de laquelle a été appliquée au moins une couche (3) d'encre en forme de motifs destinés à être vus du dessus, au moins une couche textile (4) extérieure, de laquelle font saillie, du côté extérieur, des boucles destinées à coopérer avec des crochets d'un dispositif auto-agrippant à crochets et boucles, fixée sur la face supérieure de la au moins une couche de non-tissé pour ainsi former une nappe ou un ruban en forme de stratifié, notamment pour former la "bande confort" ou "landing zone" d'une couche culotte,
**caractérisé en ce que** la au moins une couche de non-tissé et/ou l'encre sont choisies de sorte que l'encre pénètre dans la couche de non-tissé, la totalité de la couche (3) d'encre étant reçue dans la au moins une couche de non-tissé et affleure à la surface supérieure de la au moins une couche (1) de non-tissé.

2. Stratifié suivant la revendication 1, **caractérisé en ce que** l'élément non-tissé est dépourvu ou sensiblement dépourvu d'encre sur ses faces extérieure et intérieure lorsque l'on réalise sur ces deux faces le test de la bande adhésive.

3. Stratifié suivant la revendication 1 ou 2, **caractérisé en ce que** la couche (1) de non-tissé support présente une perméabilité à l'air supérieure à 1000 l/m²/s, préférentiellement supérieure à 1700 l/m²/s.

4. Stratifié non-tissé suivant l'une des revendications 1 à 3, **caractérisé en ce que** la au moins une couche de non-tissé comporte au moins deux couches empilées l'une sur l'autre, une couche supérieure, du côté extérieur, favorisant la migration en son sein de l'encre des motifs décoratifs de la couche d'impression pour ainsi recevoir l'encre, et une inférieure, du côté intérieur, moins favorable à la migration de cette encre et notamment une couche bloquant la migration de l'encre pour former les moyens de blocage de la migration de l'encre.

5. Stratifié non-tissé suivant la revendication 4, **caractérisé en ce que** la au moins une couche de non-tissé est constituée d'une couche supérieure en Spunbond et d'une couche inférieure de blocage de type Meltblown, pour ainsi former un non-tissé SM (Spunbond-Meltblown), l'épaisseur de la couche Spunbond étant suffisante pour permettre la migration de sensiblement la totalité de la couche d'encre en son sein avant que cette dernière ne soit arrêtée par la couche de type Meltblown, le non-tissé étant notamment un SMS, un SMMS, SSMMS et analogue.

6. Stratifié suivant l'une des revendications précédentes, **caractérisé en ce que** la fixation de la au moins une couche (4) extérieure textile à boucles à la au moins une couche (1) de non-tissé formant support est réalisée par soudure.

7. Stratifié suivant la revendication 6, **caractérisé en ce que** cette soudure est réalisée sans apport de matière, notamment par un calandrage thermique et/ou mécanique ou par une soudure à ultrason.

8. Stratifié suivant l'une des revendications 1 à 7, **caractérisé en ce que** la au moins une couche textile extérieure à boucles est un tricot.

9. Stratifié suivant l'une des revendications 1 à 7, **caractérisé en ce que** la au moins une couche textile extérieure à boucles est un non-tissé, notamment un non-tissé cardé consolidé, notamment un non-tissé cardé calandré.

10. Stratifié suivant l'une des revendications 1 à 9, **caractérisé en ce que** la au moins une couche textile à boucle est constituée d'un amas de fibres ou filaments non-consolidés.

11. Stratifié suivant l'une des revendications 1 à 10, **caractérisé en ce qu'**il est prévu des moyens (5) pour bloquer la migration de l'encre dans la au moins une couche de non-tissé pour la maintenir dans la partie supérieure du non-tissé, notamment en affleurement à la face extérieure du non-tissé, et ainsi assurer une bonne visibilité de l'encre à partir du côté extérieur, ou du dessus.

12. Couche culotte comportant un stratifié suivant l'une des revendications 1 à 11.

## Patentansprüche

1. Schichtstoff zur Bildung eines Streifens oder einer Lage mit Schlaufen, insbesondere des Komfort-Bands einer Höschenwindel, aufweisend:
ein Vlieselement mit wenigstens einer Vliesschicht (1), auf deren einer Oberseite wenigstens eine Tintenschicht (3) in Form von Motiven aufgebracht wurde, die dazu bestimmt sind, von oben gesehen zu werden, wenigstens einer äußeren Textilschicht (4), von der an der Außenseite Schlaufen hervorstehen, die dazu bestimmt sind, mit Haken einer Klettvorrichtung mit Haken und Schlaufen zusammenzuwirken, die auf der Oberseite der wenigstens einen Vliesschicht befestigt ist, um so eine Lage oder einen Streifen in Schichtstoffform zu bilden, insbesondere um das "Komfort-Band" oder die "Landing-Zone" einer Höschenwindel zu bilden,
**dadurch gekennzeichnet, dass** die wenigstens eine Vliesschicht und/oder die Tinte so gewählt sind, dass die Tinte in die Vliesschicht eindringt, wobei die gesamte Tintenschicht (3) in der wenigstens einen Vliesschicht aufgenommen ist und mit der oberen Fläche der wenigstens einen Vliesschicht (1) bündig ist.

2. Schichtstoff nach Anspruch 1, **dadurch gekennzeichnet, dass** das Vlieselement auf seiner Außen- und Innenseite keine oder im Wesentlichen keine Tinte aufweist, wenn auf diesen zwei Seiten der Klebebandtest durchgeführt wird.

3. Schichtstoff nach Anspruch 1 oder 2, **dadurch gekennzeichnet, dass** die Träger-Vliesschicht (1) eine Luftdurchlässigkeit größer als 1000 l/m²/s, vorzugsweise größer als 1700 l/m²/s aufweist.

4. Vliesschichtstoff nach einem der Ansprüche 1 bis 3, **dadurch gekennzeichnet, dass** die wenigstens eine Vliesschicht wenigstens zwei aufeinander gestapelte Schichten aufweist, eine obere Schicht auf der Außenseite, die die Migration der Tinte der dekorativen Motive der Druckschicht in ihrem Inneren begünstigt, um so die Tinte aufzunehmen, und eine untere auf der Innenseite, die weniger günstig für die Migration dieser Tinte ist, und insbesondere eine Schicht, die die Migration der Tinte blockiert, um die Mittel zum Blockieren der Migration der Tinte zu bilden.

5. Vliesschichtstoff nach Anspruch 4, **dadurch gekennzeichnet, dass** die wenigstens eine Vliesschicht von einer oberen Schicht aus Spunbond und einer unteren Blockierschicht vom Typ Meltblown gebildet ist, um so ein SN- (Spunbond-Meltblown-) Vlies zu bilden, wobei die Dicke der Spunbond-Schicht ausreichend ist, um die Migration im Wesentlichen der gesamten Tintenschicht in ihrem Inneren zu ermöglichen, bevor diese von der Meltblown-Schicht aufgehalten wird, wobei der Vlies insbesondere ein SMS, ein SMMS, SSMMS und dergleichen ist.

6. Schichtstoff nach einem der vorhergehenden Ansprüche, **dadurch gekennzeichnet, dass** die Befestigung der wenigstens einen äußeren Textilschicht (4) mit Schlaufen an der wenigstens einen trägerbildenden Vliesschicht (1) durch Schweißen ausgeführt ist.

7. Schichtstoff nach Anspruch 6, **dadurch gekennzeichnet, dass** dieses Schweißen ohne Materialzutrag, insbesondere durch ein thermisches und/oder mechanisches Kalandrieren oder durch ein Ultraschallschweißen, ausgeführt ist.

8. Schichtstoff nach einem der Ansprüche 1 bis 7, **dadurch gekennzeichnet, dass** die wenigstens eine äußere Textilschicht mit Schlaufen ein Trikot ist.

9. Schichtstoff nach einem der Ansprüche 1 bis 7, **dadurch gekennzeichnet, dass** die wenigstens eine äußere Textilschicht mit Schlaufen ein Vlies ist, insbesondere ein konsolidierter kardierter Vlies, insbesondere ein kalandrierter kardierter Vlies.

10. Schichtstoff nach einem der Ansprüche 1 bis 9, **dadurch gekennzeichnet, dass** die wenigstens eine äußere Textilschicht mit Schlaufen von einer Anhäufung von nicht konsolidierten Fasern oder Filamenten gebildet ist.

11. Schichtstoff nach einem der Ansprüche 1 bis 10, **dadurch gekennzeichnet, dass** Mittel (5) zum Blockieren der Migration der Tinte in der wenigstens einen Vliesschicht vorgesehen sind, um sie im oberen Abschnitt des Vlieses zu halten, insbesondere bündig mit der Außenseite des Vlieses, und so eine gute Sichtbarkeit der Tinte von der Außenseite oder von oben zu gewährleisten.

12. Höschenwindel aufweisend einen Schichtstoff nach einem der Ansprüche 1 bis 11.

## Claims

1. A laminate for forming a web or a strip with loops, in particular the landing zone of a diaper, comprising:
a non-woven element comprising at least one non-woven layer (1) to an upper face of which has been applied at least one layer (3) of ink in the form of patterns intended to be seen from above, at least one external textile layer (4) from which loops project externally in order to engage with hooks of a selffastening hook and loop device, secured to the upper face of the at least one non-woven layer so as to thus form a strip or a web in the form of a laminate, in particular to form the "landing zone" of a diaper,
**characterized in that** the at least one non-woven layer and/or the ink are chosen so that the ink penetrates into the non-woven layer, all of the layer (3) of ink being received in the at least one non-woven layer and shows on the upper surface of the at least one non-woven layer (1).

2. The laminate according to claim 1, **characterized in that** the non-woven element has no or substantially no ink on its external and internal faces when one carries out the adhesive tape test on these two faces.

3. The laminate according to claim 1 or 2, **characterized in that** the non-woven support layer exhibits permeability to air greater than 1000 l/m²/s, preferably greater than 1700 l/m²/s.

4. The laminate according to any of claims 1 to 3, **characterized in that** the at least one non-woven layer comprises at least two layers piled one over the other, an upper layer, from the outside, encouraging migration within it of the ink of the decorative patterns of the printed layer so as to thus receive the ink, and a lower layer, from the inside, less encouraging of the migration of this ink, and in particular a layer blocking the migration of the ink in order to form means for blocking migration of the ink.

5. The laminate according to claim 4, **characterized in that** the at least one non-woven layer is formed by an upper layer of Spunbond and a lower blocking layer of the Meltblown type so as to thus form an SM (Spunbond-Meltblown) non-woven fabric, the thickness of the Spunbond layer being sufficient to allow the migration of substantially all of the layer of ink within it before the latter is stopped by the Meltblown-type layer, the non-woven fabric being in particular an SMS, an SMMS, an SSMMS or the like.

6. The laminate according to any of the preceding claims, **characterized in that** the securing of the at least one external textile layer (4) with loops to the at least one non-woven layer (1) forming a support is achieved by welding.

7. The laminate according to claim 6, **characterized in that** this welding is achieved without the use of any substance, in particular it is achieved by thermal and/or mechanical calendering or by ultrasonic welding.

8. The laminate according to any of claims 1 to 7, **characterized in that** the at least one external textile layer with loops is a knit.

9. The laminate according to any of claims 1 to 7, **characterized in that** the at least one external textile layer with loops is a non-woven fabric, in particular a consolidated and carded non-woven fabric, more particularly a calendered and carded non-woven fabric.

10. The laminate according to any of claims 1 to 9, **characterized in that** the at least one textile layer with loops is formed by a cluster of unstrengthened fibers or filaments.

11. The laminate according to any of claims 1 to 10, **characterized in that** means (5) are provided for blocking the migration of ink into the at least one non-woven layer so as to keep it in the upper part of the non-woven fabric, in particular showing on the surface on the external face of the non-woven fabric, and thus ensuring good visibility of the ink from the outside or from above.

12. A diaper comprising a laminate according to any of claims 1 to 11.
